# EUROPEAN PATENT SPECIFICATION

(11) **EP 1 946 230 B1**
(45) Date of publication and mention of the grant of the patent: **19.10.2016**
(21) Application number: 05798706.7
(22) Date of filing: 09.11.2005
(51) Int. Cl.: G06F 19/22, G06F 19/28

(54) **COMPUTER-IMPLEMENTED METHOD AND COMPUTER SYSTEM FOR IDENTIFYING ORGANISMS**
RECHNERIMPLEMENTIERTES VERFAHREN UND RECHNERSYSTEM ZUR IDENTIFIZIERUNG VON ORGANISMEN
PROCEDE ET SYSTEME INFORMATIQUES PERMETTANT D'IDENTIFIER DES ORGANISMES

(43) Date of publication of application: 23.07.2008
(73) Proprietor: Smartgene GmbH, 6300 Zug (CH)
(72) Inventor: EMLER, Stefan, 8032 Zürich (CH)
(74) Representative: Müller-Boré & Partner Patentanwälte PartG mbB
(86) International application number: PCT/CH2005/000664
(87) International publication number: WO 2007/053962

(56) References cited:
- WO-A-03/069534
- HARMSEN DAG ET AL: "RIDOM: Ribosomal differentiation of medical micro-organisms database" NUCLEIC ACIDS RESEARCH, vol. 30, no. 1, 1 January 2002 (2002-01-01), pages 416-417, XP002365651 ISSN: 0305-1048
- CHAN MAN-SUEN ET AL: "Database-driven multi locus sequence typing (MLST) of bacterial pathogens" BIOINFORMATICS (OXFORD), vol. 17, no. 11, November 2001 (2001-11), pages 1077-1083, XP002364900 ISSN: 1367-4803
- HARMSEN D ET AL: "Typing of Methicillin-Resistant Staphylococcus aureus in a University Hospital Setting by Using Novel Software for spa Repeat Determination and Database Management" JOURNAL OF CLINICAL MICROBIOLOGY, vol. 41, no. 12, December 2003 (2003-12), pages 5442-5448, XP002364892
- ROZANOV MIKHAIL ET AL: "A web-based genotyping resource for viral sequences" NUCLEIC ACIDS RESEARCH, vol. 32, no. Suppl. 2, 1 July 2004 (2004-07-01), pages W654-W659, XP002364901 ISSN: 0305-1048

## Description

The present invention relates to a computer-implemented method and a computer system for identifying organisms. Specifically, the present invention relates to a computer-implemented method and a computer system for identifying organism types from a target gene sequence. The present invention relates also to a computer program product for controlling the computer-based system such that the system executes the method of identifying organism types from the target gene sequence.

Medical diagnostics increasingly rely on analysis of genetic targets of humans or microorganisms. Typically, this analysis is based on comparison of an individual target gene sequence to reference sequences from a reference database. The closest matching reference sequence is retrieved from the reference database. Thus, for identifying organism types from a target gene sequence, the conventional methods and systems compare and retrieve reference sequences with respect to their similarity with the target sequence. Conventionally, similarity is determined from overall matches over the longest common segment of target and reference sequence. Taken into account is the number of mismatches in a segment regardless of their positions. For example, in microbiology, the Ribosomal Database Project (RDP-II), accessible on http://rdp.cme.msu.edu/, the Ribosomal Differentiation of Microorganisms (RIDOM™), accessible on http://www.ridom.de/, and the European Ribosome RNA Database, accessible on http://www.psb.ugent.be/rRNA/, offer classification services based on bacterial 16S rDNA (ribosomal Deoxyribonucleic Acid) sequences. In these systems, used are methods that include the following steps: (i) match the target sequence against a reference database of 16S rDNA sequences, producing a set of closely matching sequences; (ii) build a multiple sequence alignment (MSA) from the target sequence and this set of reference sequences; (iii) extract a distance matrix from the MSA, and use this matrix to build an evolutionary tree; (iv) place the query sequence on the tree, and visualize the resulting relationships. However, these systems do not discriminate between inter-sequence differences that could be trivial in origin, e.g. due to sequencing errors or biologically unimportant variations, and those found in positions that are known to be diagnostic of inter-strain or inter-species differences. Consequently, in these systems, sequencing errors or random mutations at regions which are not significant have the same impact on sequence ranking as mismatches in certain variable regions related to genus or species, for example. As positions of these variable regions are not known before the organism type (e.g. genus, species, sub-type, variant or clade) of a given sample is identified, the sequence-comparison-based methodology is very user-dependent and requires a level of expertise one does not easily find in diagnostic labs.

It is an object of this invention to provide a computer-implemented method and a computer system for identifying organism types from a target gene sequence, an organism type including entity, genus, species, sub-type, variant and/or clade associated with the organism, which system and method do not have the disadvantages of the prior art. In particular, it is an object of the present invention to provide a computer-implemented method and a computer system for identifying organism types from a target gene sequence, which system and method discriminate between trivial and significant inter-sequence differences. References made herein to the term "organism" or "organism type" shall be understood to include the terms "entity", "genus", "species", "sub-type", "group", "strain", "variant" and/or "clade" in the context of taxonomic classification.

According to the present invention, the above-mentioned objects are achieved particularly through the features of the independent claims. In addition, further advantageous embodiments follow from the dependent claims and the description.

According to the present invention, the above-mentioned objects are particularly achieved in that, for identifying organism types from a target gene sequence, selected automatically from a database is a selected sequence profile having a highest correlation with the target gene sequence based on position-specific information of the related organism-type specific profile. The sequence profile is selected from a plurality of organism type-specific profiles in the database, each profile defining informative sequence regions for differentiating individual organism types and including said position-specific information related to more than one associated reference sequences of a specific organism type. Preferably, the type-specific profiles include genus-specific or group-specific profiles; moreover, the type-specific profiles may include species-specific, sub- type-specific, variant-specific, and/or clade-specific profiles. Reference sequences, associated with the selected sequence profile, are retrieved automatically from the database. The target gene sequence is compared automatically to the reference sequences and comparison results, related to the informative sequence regions, are weighted automatically using weighting factors associated with the informative sequence regions defined in the selected sequence profile. Subsequently, from the reference sequences, determined is an organism type-specific reference sequence having a best match with the target gene sequence, the best match being determined based on the comparison results weighted for the informative sequence regions. The type-specific reference sequence having the best match with the target gene sequence, considering the weighted comparison results, is selected automatically or set as a top entry in a sorted list. Weighting for the informative sequence regions the comparison results makes it possible to identify the organism type from the target gene sequence while discriminating between trivial and significant inter-sequence differences. The results obtained through profile search and weighted alignment will provide a measurement reflecting correct assignment of organism type in bacteriology, mycology and virology. Consequently, the assignment of organism types, e.g. bacterial and fungal species or viral subtypes, is improved. Organism types are assigned on the basis of not just statistical criteria but also on the basis of biologically relevant profiles. Consequently, more reliable results are derived for sequence analysis in an easy to use routine set-up. Generally, the time needed to produce results is shortened and the treatment of patients will benefit from more rapid and precise results.

In an embodiment, the comparison results include a number of differences and/or correspondences in nucleotide codes of each of the reference sequences when compared to the target gene sequence (nucleotide codes also including IUPAC (International Union of Pure and Applied Chemistry) codes). Weighting the comparison results includes determining for each reference sequence a weighted number of differences and/or correspondences by multiplying with a weighting factor the number of differences and/or correspondences related to the informative sequence regions. Furthermore, stored is a list of the reference sequences, sorted by the weighted number of differences and/or correspondences of the respective reference sequence.

In a preferred embodiment, the target gene sequence and the reference sequences associated with the selected sequence profile are assessed for new informative sequence regions for the selected sequence profile. Moreover, the selected profile is adapted by storing a new informative sequence region as a part of the selected sequence profile. Refining the sequence profile with newly identified informative sequence regions make it possible to consider evolutionary aspects of organisms, e.g. evolutionary relationships between species and strains. Continuous adaptation of sequence profiles help to adjust phylogenetic and ultimately taxonomic annotations and thus will provide important information to microbiologists and physicians with regard to the pathogenicity and epidemiology of unknown ormisclassified microorganisms.

In an embodiment, assessing the target gene sequence and the reference sequences includes aligning the target gene sequence and the reference sequences associated with the selected sequence profile, and identifying new informative sequence regions. New informative sequence regions are determined by identifying nucleotide codes corresponding at a same sequential position in at least a defined number of the target gene sequence and reference sequences.

Preferably, the organism type-specific sequence profiles stored in the database are determined by aligning type-specific gene sequences, by creating consensus sequences per organism type, by identifying the informative regions that enable differentiating the individual organism types, by adding sequence annotation information, and by defining the type-specific sequence profiles based on the informative regions.

In an embodiment, the organism type-specific sequence profiles stored in the database include genus-specific or group-specific sequence profiles. The genus-specific or group- specific profiles are determined by aligning genus-specific or group-specific gene sequences, by creating consensus sequences per organism type, by identifying the informative regions that enable differentiating the individual organism types, and by defining the genus-specific or group-specific sequence profiles based on the informative regions.

In a further embodiment, the target gene sequence is proofread based on the selected sequence profile by comparing the target gene sequence to the reference sequences associated with the selected sequence profile. For differences of nucleotide codes, located in informative sequence regions, it is assessed whether the differences indicate another organism type. Adaptation of the selected sequence profile is initiated for differences assessed to indicate another organism type. Automatic proofreading based on the selected sequence profile makes it possible to proofread the target gene sequence while discriminating between trivial and significant inter-sequence differences.

Preferably, the target gene sequence is received by a server from a user via a telecommunications network. Furthermore, the organism type of the target gene sequence, defined by the organism type-specific reference sequence, is transmitted by the server via the telecommunications network to a user interface. Implementing the process on a network-based server makes it possible to provide efficiently (in terms of performance and financial costs) automatic identification of organism types from a target gene sequence as a centralized service, available to a plurality of users connected to the telecommunications network. Using a server-based technology for identifying organism types from a target gene sequence makes it possible for a user to use its own computer equipment without having to install any software or hardware. In the networked database, type-specific profiles can be added and improved continuously on the basis of target sequences supplied over the network by users. In addition, the reference sequence database, the software application, as well as any software tools can be updated online without any disturbance to users. Moreover, the network-based server enables exchange and sharing of data between distant expert institutes as well as assessment of database entries representing organism types, e.g. bacterial and fungal species or viral subtypes, with respect to their taxonomic classification. Thus, the network- based server makes it possible for experts to re-evaluate and validate reference data sets for bacteria, mycobacteria, fungi, and viruses.

In addition to a computer-implemented method and a computer system for identifying organism types from a target gene sequence, the present invention also relates to a computer program product including computer program code means for controlling one or more processors of the computer system such that the system executes the method of identifying organism types from a target gene sequence. Particularly, a computer program product including a computer readable medium containing therein the computer program code means (e.g. programmed software modules, as described later in more detail).

The present invention will be explained in more detail, by way of example, with reference to the drawings in which:
Figure 1 shows a block diagram illustrating schematically an exemplary configuration of a computer system for practicing embodiments of the present invention, said configuration comprising a server with a database, and said configuration being connected to a data entry terminal via a telecommunications network.
Figure 2 shows a flow diagram illustrating an example of a sequence of steps for establishing a sequence profile for a target organism.
Figure 3 shows a flow diagram illustrating an example of a sequence of steps executed according to the present invention for identifying an organism type from a target gene sequence.
Figure 4 shows a flow diagram illustrating an example of a sequence of steps executed according to the present invention for adapting sequence profiles based on a target gene sequence.

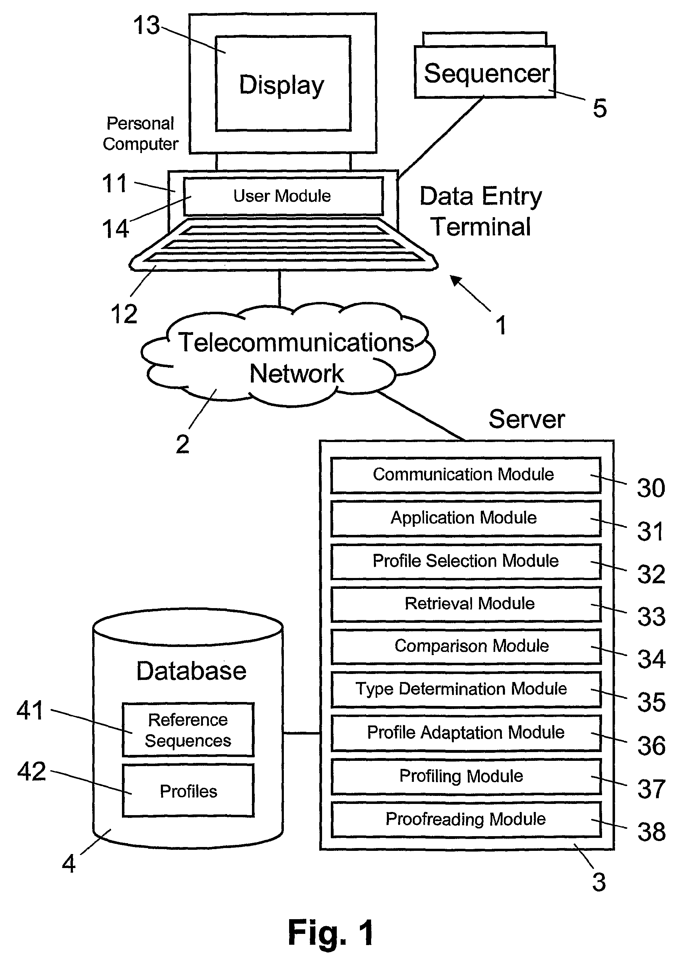

In Figure 1, reference numeral 1 refers to a data entry terminal. As illustrated in Figure 1, the data entry terminal 1 includes a personal computer 11 with a keyboard 12 and a display monitor 13. As is illustrated schematically, in an embodiment, the personal computer 11 includes a user module 14 implemented as a programmed software module, for example an executable program applet that is downloaded from server 3 via telecommunications network 2.

Connected to the personal computer 11 is a conventional sequencer 5, which provides the personal computer 11 with sequence data of DNA (Deoxyribonucleic Acid) fragments. For example, the fragment sequence data includes sequence signals and associated information (e.g. peak values) of the DNA fragments, each sequence signal including signals of the four nucleotide types Adenine, Cytosine, Guanine, and Thymine (A, C, G, T). Generally, the terms "gene sequence", "target sequence", or "reference sequence" are used herein to refer to a sequence of nucleotide codes, i.e. a sequence of codes, each representing one of the nucleotide types. The sequences are related to bacteria, fungi, microfungi, and viruses, for example.

As is illustrated in Figure 1, the data entry terminal 1 is connected to server 3 through telecommunications network 2. Preferably, the telecommunications network 2 includes the Internet and/or an Intranet, making server 3 accessible as a web server through the World Wide Web or within a separate IP-network, respectively. Telecommunications network 2 may also include another fixed network, such as a local area network (LAN) or an integrated services digital network (ISDN), and/or a wireless network, such as a mobile radio network (e.g. Global System for Mobile communication (GSM) or Universal Mobile Telephone System (UMTS)), or a wireless local area network (WLAN). In a local embodiment, data entry terminal 1 is connected directly to server 3, or data entry terminal 1 and server 3 are implemented on the same computer, e.g. a PC.

As is illustrated schematically in Figure 1, server 3 is connected to database 4. Server 3 may include one or more computers, each having one or more processors. The database 4 may be implemented on a computer shared with server 3 or on a separate computer.

The server 3 includes different functional modules, namely a communication module 30, an application module 31, a profile selection module 32, a retrieval module 33, a comparison module 34, a type determination module 35, a profile adaptation module 36, a profiling module 37, and a proofreading module 37. The communication module 30 includes conventional hardware and software elements configured for exchanging data via telecommunications network 2 with a plurality of data entry terminals 1. The application module 31 is a programmed software module configured to provide users of the data entry terminal 1 with a user interface. Preferably, the user interface is provided through a conventional Internet browser such as Microsoft Explorer or Mozilla. Alternatively, the user interface is generated by user module 14. For example, application module 31 transmits a copy of the user module 14 via telecommunications network 2 to the personal computer 11 of the user. The user module 14 is installed and activated on the personal computer 11. When activated, user module 14 controls a processor of the personal computer 11 such that it generates the user interface on display 13. The profile selection module 32, the retrieval module 33, the comparison module 34, the type determination module 35, the profile adaptation module 36, the profiling module 37, and the proofreading module 37 are programmed software modules executing on a computer of server 3.

As is illustrated schematically in Figure 1, database 4 includes reference sequences 41 and associated sequence profiles 42 for different types of organisms. The profiles 42 are stored assigned to the reference sequences 41. Typically, more than one reference sequence 41 is assigned to a sequence profile 42. The profiles 42 define informative sequence regions for differentiating individual organisms, i.e. the profiles 42 include position-specific information related to one or more associated reference sequence 41 of a specific type of organism, i.e. a specific genus, species, sub-type, variant, and/or clade. The position-specific information is assigned to one specific position or to a range of multiple positions in a sequence. The range is defined, for example, by a start and an end position in the reference sequence or by a start position and a length (i.e. number of sequence positions). Alternatively, position-specific information may also be represented by means of Hidden Markov Models (HMM). While they are not identical, profiles 42 and HMM are sufficiently similar so that they will be used interchangeably in the present context. The sequence profiles 42 summarise the information contained in a multiple sequence alignment: the position-specific annotations document, for each position (column) in the alignment, the probability of finding a given residue (pattern) at that position, the cost of opening a gap in the alignment before or after this position ("open gap cost"), and the probabilities of finding particular residues immediately preceding or following the current position. As is illustrated in Table 1, in an embodiment, the profiles 42 are provided with additional information, e.g. the weight assigned to individual positions when calculating alignment scores, or structural parameters associated with ranges of positions.

**Table 1**

| | | | | |
|---|---|---|---|---|
| Sequence Profile | | | | Assigned reference gene sequence(s) |
| Profile identifier | Position-specific information | | | |
| | Region/position specification | Annotation | Weighting factor | |

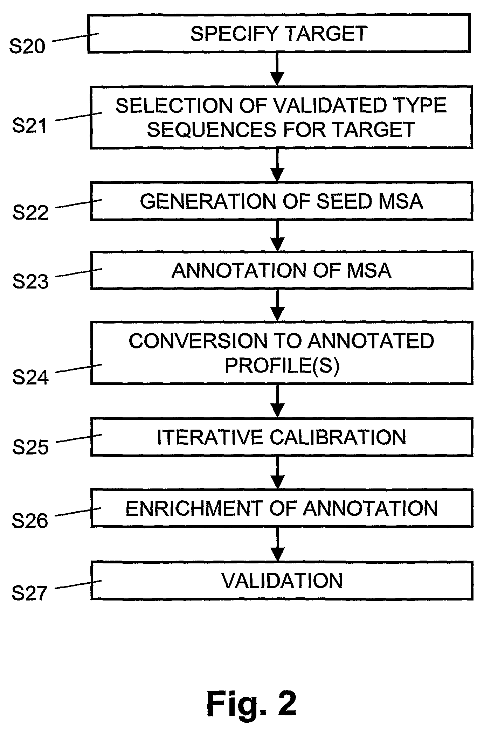

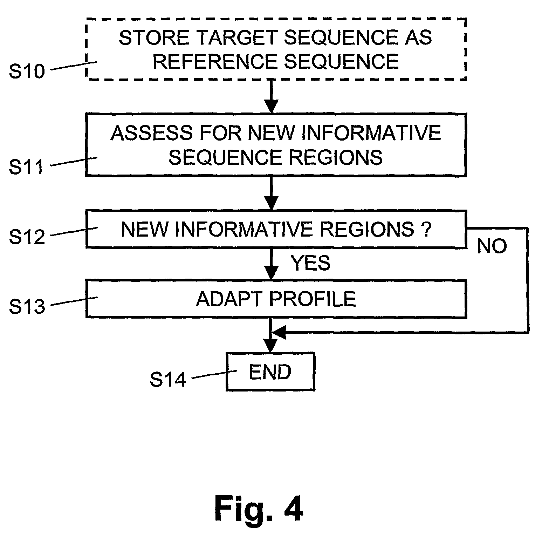

The initial profiles 42 are established and stored by profiling module 37, as will be described below with reference to Figure 2. Further refinement and adaptation of the profiles is provided by profile adaptation module 36, as will be described later with reference to Figure 4.

As is illustrated in Figure 2, in step S20, a target organism or group of target organisms is specified. Preferably, the target organism is defined by a specific genus or species. However, one skilled in the art will understand that the target organism may be further defined by a sub-type, variant, and/or clade. The target is specified by a user through a user interface visualized on display 13 or on another data entry terminal connected to server 3. For example, the target is selected from a drop down list or entered in a data entry field. Alternatively, the target may also be selected automatically by the profiling module 37 from a list of possible targets.

In step S21, validated type sequences are selected for the target specified in step S20. For example, the validated gene sequences are selected automatically by the profiling module 37 from the reference sequences 41 in database 4. However, at least inifiially, the validated gene sequences are either retrieved from a validated reference database or selected and entered by an expert using data entry terminal 1. The validated type sequences for the specified target cover all the known variable positions, i.e. all the informative sequence regions (positions) that are known to be diagnostic of inter-strain or inter-species differences and thus indicative of organism types (including genus, species, sub-type, variant, and/or clade).

In step S22, using the profiling module 37, a seed MSA (multiple sequence alignment) is generated from the validated type sequences selected in step S21. Particularly, using the profiling module 37 the type sequences are aligned and a consensus sequence is created for the respective organism type.

In step S23, identified are informative regions that enable differentiating individual organism types. Using the profiling module 37, the MSA generated in step S22 is provided with annotations for secondary structures (3' and 5' pairing regions), and for positions known to be diagnostic for inter-strain or inter-species differences in the target organism, i.e. positions known to be indicative of organism types (including genus, species, sub-type, variant, and/or clade).

In step S24, the profiling module 37 converts the annotated MSA to one or more annotated profiles and stores these profiles 42 in database 4.

In step S25, using the profiling module 37, the profiles 42 stored in step S24 are calibrated iteratively. Iterative calibration is achieved by using the profile(s) to search a collection of reference sequences known (validated by experts) to belong or not to belong to the respective organism type, i.e. genus, species, sub-type, variant, and/or clade.

In step S26, using the profiling module 37, the annotation of the profiles 42 are enriched by including positions that discriminate between the target organism and other genera.

In step S27, the annotated profiles are validated by experts in the field and through statistics on available samples sequences. For validation purposes, the profiles 42 stored in database 4 are made available to the experts through server 3 and telecommunications network 2. For example, the validated profiles are provided with an indicator, or an electronic certificate or signature in database 4.

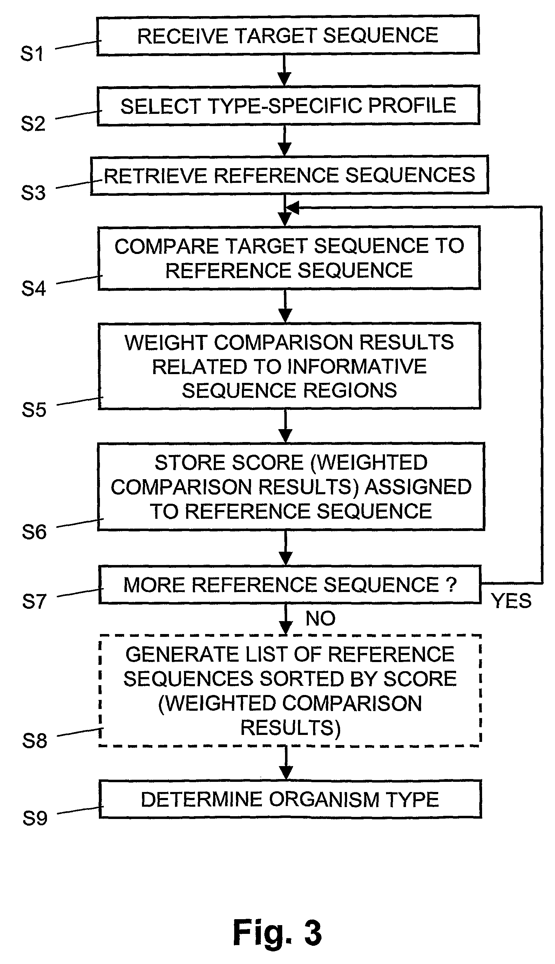

In the following paragraphs, the identification of an organism type from a target gene sequence is described with reference to Figure 3.

In step S1, a target gene sequence is received by the communication module 30 via telecommunications network 2. The target gene sequence is provided by data entry terminal 1. For example, the target gene sequence is stored in personal computer 11 or generated from sequence data of DNA fragments provided by sequencer 5. Preferably, the target gene sequence is defined by a user through a user interface visualized on display 13 by application module 31 or by user module 14. Subsequently, the process for identifying the organism type is initiated automatically. Alternatively, the process is initiated by the user activating a control element such as a graphical button in the user interface.

In step S2, the profile selection module 32 determines in database 4 the sequence profile 42 having the highest correlation with the target gene sequence received in step S1. The degree of correlation between the target gene sequence and the sequence profiles 42 is determined based on the position-specific information contained in the sequence profiles 42, i.e. the profile selection module 32 uses the profile annotations on informative sequence regions to select the profile 42 having the best match with the target gene sequence. Preferably, the best matching sequence profile is determined by applying for each profile its position-specific weighting factors to deviations and/or correspondences of the target sequence from/with the profile.

In step S3, the retrieval module 33 loads from database 4 the reference gene sequences 41 associated with the sequence profile 42 selected in step S2.

In step S4, the comparison module 34 compares the target gene sequence, received in step S1, to one of the reference gene sequences, retrieved in step S3. Furthermore, the comparison module 34 weights the comparison results with weighting factors associated with the sequence profile, particularly, weighting factors associated with the informative sequence regions. Consequently, comparison results related to a first sequence region may be weighted with another weighting factor than comparison results related to another second sequence region. Thus, the comparison module 34 weights the number of differences and/or the number of correspondences, between the target gene sequence and the respective reference gene sequence, using weighting factors associated with the informative sequence regions outlined in the profile 42.

In step S6, the comparison module 34 stores a score, indicative of the matching level, assigned to the respective reference gene sequence. The score is based on the weighted comparison results. For example, comparison module 34 stores a score based on the weighted number of differences and/or correspondences.

In step S7, the application module 31 checks whether or not there are further reference gene sequences assigned to the selected profile that need to be processed. If there are more reference sequences to be processed, processing continues in step S4. Otherwise, if all reference sequences assigned to the selected profile have been processed, processing continues in step S8.

In optional step S8, the type determination module 35 generates a full or partial list of the reference sequences assigned to the selected profile. The list is sorted by the score assigned to the reference sequences. For example, the list (with its entries and assigned scores) is transmitted via telecommunications network 2 to the data entry terminal 1 where it is shown to the user on display 13.

In step S9, the type determination module 35 determines the type-specific reference gene sequence having the best match with the target gene sequence. The type determination module 35 determines the type-specific reference gene sequence based on the assigned scores, i.e. the weighted comparison results, e.g. the weighted number of differences and/or correspondences assigned to the reference sequences retrieved in step S3. For example, the type-specific reference gene sequence is defined by the lowest weighted number of differences and/or the highest weighted number of correspondences. The type information associated with the type-specific reference gene sequence is selected to define the organism type of the target gene sequence. Thus, the organism type of the target gene sequence is defined by the genus, species, sub-type, variant, and/or clade associated with the type-specific reference gene sequence. Preferably, the organism type and its assigned score are transmitted by the communication module 30 via telecommunications network 2 to the data entry terminal 1 where the organism type and its assigned score are shown to the user on display 13.

As is illustrated in Figure 4, in an embodiment, subsequent to steps S1 to S9, the target gene sequence received in step S1 is used for refining the sequence profile 42 selected in step S2. In optional step S10, the profile adaptation module 36 stores the target gene sequence as a reference gene sequence in database 4, assigned to the profile 42 selected in step S2. In an embodiment, the target gene sequence is stored as a sample gene sequence in database 4. Refinement of the sequence profile 42 is triggered, for example, by a defined number of stored samples and/or statistics on stored samples and reference sequences.

In step S11, the profile adaptation module 36 assesses the new reference gene sequence from step S10, or one or more sample gene sequences stored in step S10, for new informative sequence regions. Particularly, the profile adaptation module 36 determines whether the reference gene sequences (and possibly sample gene sequences) assigned to the respective sequence profile 42 have informative sequence regions that are not yet included in the sequence profile but indicate an organism type, i.e. a genus, species, sub-type, variant, and/or clade. In essence, the profile adaptation module 36 determines sequence positions or regions that have a correlation across the respective reference gene sequences (and possibly sample gene sequences) exceeding a defined correlation threshold. For example, the profile adaptation module 36 aligns the new reference sequence (and possibly the sample gene sequences) and the reference sequences related to the selected profile, and identifies new informative sequence regions by identifying nucleotide codes corresponding at the same sequential position in at least a defined number of the new reference sequence (and possibly sample gene sequences) and reference sequences.

In step S12, the profile adaptation module 36 determines whether or not there is one or more new informative sequence region. If there are new informative sequence regions, processing continues in step S13. Otherwise, processing of the target gene sequence ends in step S14.

In step S13, based on the new informative sequence region(s) determined in step S11, the profile adaptation module 36 adapts the sequence profile selected in step S2. Thus, the sequence profile selected in step S2 is refined by adding the new informative sequence region(s) to the sequence profile. Subsequently, the refined sequence profile may be subject to iterative calibration and validation, as described in the context of steps S25 and S27.

The proofreading module 38 is configured to support proofreading of or proofread automatically the target gene sequence, received in step S1, based on the sequence profile, selected in step S2. To support a user in proofreading the target sequence, the proofreading module 38 indicates in a user interface any informative sequence regions that enable differentiation of organism type. Preferably, this indication is provided in an alignment of target sequence, reference sequence(s) and/or a consensus sequence, displayed in the user interface, by highlighting visually the informative sequence regions. To provide automatic proofreading, for differences arising from nucleotide codes located in informative sequence regions, indicative of organism types, the proofreading module 38 calculates values for the probability that the difference is due to an error in the target sequence or that the difference indicates another organism type. Furthermore, the proofreading module 38 applies threshold values to the calculated probability values and corrects automatically an error (e.g. in the consensus sequence), or inserts (into the consensus sequence) a special code indicating ambiguity, for example an IUPAC (Infiernational Union of Pure and Applied Chemistry) code, or triggers an adaptation of the sequence profile, as described above with reference to Figures 2 and 4.

In describing representative embodiments of the invention, the specification may have presented the method and/or process of the invention as a particular sequence of steps. However, to the extent that the method or process does not rely on the particular order of steps set forth herein, the method or process should not be limited to the particular sequence of steps described. As one of ordinary skill in the art would appreciate, other sequences of steps may be possible. Therefore, the particular order of the steps set forth in the specification should not be construed as limitations on the claims.

The foregoing disclosure of the embodiments of the invention has been presented for purposes of illustration and description. It is not intended to be exhaustive or to limit the invention to the precise forms disclosed. Many variations and modifications of the embodiments described herein will be apparent to one of ordinary skill in the art in light of the above disclosure. The scope of the invention is to be defined only by the claims appended hereto, and by their equivalents. Particularly, in addition to the sectors of bacteriology, mycology and virology, the present inventions can also be applied in any other sectors where sequence similarity searches are involved, e.g. in human and veterinary diseases and disease predispositions (e.g. cancer), in infectious diseases such as HBV, HIV etc., as well as in typing of animals, humans, plants, microorganisms and viruses.

## Claims

1. A computer-implemented method of identifying organism types from a target gene sequence, comprising:
receiving the target gene sequence;
selecting automatically in a database (4) from a plurality of organism type-specific sequence profiles (42), each sequence profile (42) defining informative sequence regions for differentiating individual organism types and including position-specific information related to more than one associated reference sequences (41) of a specific organism type, a selected sequence profile (42) having a highest correlation with the target gene sequence based on said position-specific information of the related organism type-specific profile;
retrieving automatically from the database (4) reference sequences (41) associated with the selected sequence profile (42);
comparing automatically the target gene sequence to the reference sequences (41) and weighting automatically comparison results related to the informative sequence regions using weighting factors associated with the informative sequence regions defined in the selected sequence profile;
determining from the reference sequences (41) an organism type-specific reference sequence (41) having a best match with the target gene sequence, the best match being determined based on the comparison results weighted for the informative sequence regions; and
assigning to the target gene sequence an organism type based on the best matched organism type-specific reference sequence.

2. The method according to claim 1, wherein the comparison results include a number of differences in nucleotide codes of each of the reference sequences (41) when compared to the target gene sequence; wherein weighting the comparison results includes determining for each reference sequence (41) a weighted number of differences by multiplying with a weighting factor the number of differences related to the informative sequence regions; and wherein the method further includes storing a list of the reference sequences (41), the list being sorted by the weighted number of differences of the respective reference sequence (41).

3. The method according to one of claims 1 or 2, wherein the comparison results include a number of correspondences in nucleotide codes of each of the reference sequences (41) when compared to the target gene sequence; wherein weighting the comparison results includes determining for each reference sequence (41) a weighted number of correspondences by multiplying with a weighting factor the number of correspondences related to the informative sequence regions; and wherein the method further includes storing a list of the reference sequences (41), the list being sorted by the weighted number of correspondences of the respective reference sequence (41).

4. The method according to one of the claims 1 to 3, wherein the target gene sequence and the reference sequences (41) associated with the selected sequence profile (42) are assessed for new informative sequence regions for the selected sequence profile (42); and wherein the selected sequence profile (42) is adapted by storing a new informative sequence region as a part of the selected sequence profile (42).

5. The method according to claim 4, wherein assessing the target gene sequence and the reference sequences (41) includes aligning the target gene sequence and the reference sequences (41) associated with the selected sequence profile (42), and identifying the new informative sequence regions by identifying nucleotide codes corresponding at a same sequential position in at least a defined number of the target gene sequence and reference sequences (41).

6. The method according to one of the claims 1 to 5, wherein the organism type-specific sequence profiles (42) stored in the database (4) are determined by aligning organism type-specific gene sequences, by creating consensus sequences per organism type, by identifying the informative regions that enable differentiating the individual organism types, and by defining the organism type-specific sequence profiles (42) based on the informative regions.

7. The method according to claim 6, wherein the organism type-specific sequence profiles (42) stored in the database (4) include genus-specific or group-specific sequence profiles (42), and wherein the genus-specific or group-specific sequence profiles (42) are determined by aligning genus-specific or group-specific gene sequences, by creating consensus sequences per organism type, by identifying the informative regions that enable differentiating the individual organism types, and by defining the genus-specific or group-specific sequence profiles (42) based on the informative regions.

8. The method according to one of the claims 1 to 7, wherein the target gene sequence is proofread based on the selected sequence profile (42) by comparing the target gene sequence to the reference sequences (41) associated with the selected sequence profile (42), by assessing for differences of nucleotide codes, located in informative sequence regions, whether the differences indicate another organism type, and by initiating adaptation of the selected sequence profile (42) for differences assessed to indicate another organism type.

9. The method according to one of the claims 1 to 8, wherein the target gene sequence is received by a server (3) from a user via a telecommunications network (2); and wherein the organism type of the target gene sequence, defined by the organism type-specific reference sequence (41), is transmitted by the server (3) via the telecommunications network (2) to a user interface.

10. A computer system for identifying organism types from a target gene sequence, the system comprising:
a database (4) comprising a plurality of organism type-specific sequence profiles (42), each sequence profile (42) defining informative sequence regions for differentiating individual organism types and including position-specific information related to more than one associated reference sequences (41) of a specific organism type;
a profile (42) selection module (32) configured to select automatically from said sequence profiles (42) a selected sequence profile (42) having a highest correlation with the target gene sequence based on said position-specific information of the related organism type-specific profile;
a retrieval module (33) configured to retrieve automatically from the database (4) reference sequences (41) associated with the selected sequence profile (42);
a comparison module (34) configured to compare automatically the target gene sequence to the reference sequences (41) and weighting automatically comparison results related to the informative sequence regions using weighting factors associated with the informative sequence regions defined in the selected sequence profile;
a type determination module (35) configured to determine from the reference sequences (41) an organism type-specific reference sequence (41) having a best match with the target gene sequence, the best match being determined based on the comparison results weighted for the informative sequence regions; and
assigning to the target gene sequence an organism type based on the best matched organism type-specific reference sequence.

11. The system according to claim 10, wherein the comparison results include a number of differences in nucleotide codes of each of the reference sequences (41) when compared to the target gene sequence; wherein the comparison module (34) is configured to determine for each reference sequence (41) a weighted number of differences by multiplying with a weighting factor the number of differences related to the informative sequence regions; and wherein the type determination module (35) is configured to store a list of the reference sequences (41), the list being sorted by the weighted number of differences of the respective reference sequence (41).

12. The system according to one of claims 10 or 11, wherein the comparison results include a number of correspondences in nucleotide codes of each of the reference sequences (41) when compared to the target gene sequence; wherein the comparison module (34) is configured to determine for each reference sequence (41) a weighted number of correspondences by multiplying with a weighting factor the number of correspondences related to the informative sequence regions; and wherein the type determination module (35) is configured to store a list of the reference sequences (41), the list being sorted by the weighted number of correspondences of the respective reference sequence (41).

13. The system according to one of the claims 10 to 12, further comprising a profile (42) adaptation module (36) configured to assess the target gene sequence and the reference sequences (41) associated with the selected sequence profile (42) for new informative sequence regions for the selected sequence profile (42), and to adapt the selected sequence profile (42) by storing a new informative sequence region as a part of the selected sequence profile (42).

14. The system according to claim 13, wherein the profile (42) adaptation module (36) is configured to align the target gene sequence and the reference sequences (41) associated with the selected sequence profile (42), and to identify the new informative sequence regions by identifying nucleotide codes corresponding at a same sequential position in at least a defined number of the target gene sequence and reference sequences (41).

15. The system according to one of the claims 10 to 14, further comprising a profiling module (37) configured to determine the organism type-specific sequence profiles (42) stored in the database (4) by aligning organism type-specific gene sequences, by creating consensus sequences per organism type, by identifying the informative regions that enable differentiating the individual organism types, and by defining the organism type-specific sequence profiles (42) based on the informative regions.

16. The system according to claim 15, wherein the organism type-specific sequence profiles (42) stored in the database (4) include genus-specific or group-specific sequence profiles (42), and wherein the profiling module (37) is configured to determine the genus-specific or group-specific sequence profiles (42) by aligning genus-specific or group-specific gene sequences, by creating consensus sequences per organism type, by identifying the informative regions that enable differentiating the individual organism types, and by defining the genus-specific or group-specific sequence profiles (42) based on the informative regions.

17. The system according to one of the claims 10 to 16, further comprising a proof reading module (38) configured to proofread the target gene sequence based on the selected sequence profile (42) by comparing the target gene sequence to the reference sequences (41) associated with the selected sequence profile (42), to assess for differences of nucleotide codes, located in informative sequence regions, whether the differences indicate another organism type, and to initiate adaptation of the selected sequence profile (42) for differences assessed to indicate another organism type.

18. The system according to one of the claims 10 to 17, further comprising a communication module (30) configured to receive the target gene sequence from a user via a telecommunications network (2), and to transmit the organism type of the target gene sequence, defined by the organism type-specific reference sequence (41), via the telecommunications network (2) to a user interface.

19. A computer program product adapted to perform the method of claim 1.

20. The computer program product according to claim 19, comprising further computer program code means for controlling the processors of the computer system, such that the system includes in the comparison results a number of differences in nucleotide codes of each of the reference sequences (41) when compared to the target gene sequence; determines for each reference sequence (41) a weighted number of differences by multiplying with a weighting factor the number of differences related to the informative sequence regions; and stores a list of the reference sequences (41), the list being sorted by the weighted number of differences of the respective reference sequence (41).

21. The computer program product according to one of claims 19 or 20, comprising further computer program code means for controlling the processors of the computer system, such that the system includes in the comparison results a number of correspondences in nucleotide codes of each of the reference sequences (41) when compared to the target gene sequence; determines for each reference sequence (41) a weighted number of correspondences by multiplying with a weighting factor the number of correspondences related to the informative sequence regions; and stores a list of the reference sequences (41), the list being sorted by the weighted number of correspondences of the respective reference sequence (41).

22. The computer program product according to one of claims 19 to 21, comprising further computer program code means for controlling the processors of the computer system, such that the system assesses the target gene sequence and the reference sequences (41) associated with the selected sequence profile (42) for new informative sequence regions for the selected sequence profile (42); and adapts the selected sequence profile (42) by storing a new informative sequence region as a part of the selected sequence profile (42).

23. The computer program product according to claim 22, comprising further computer program code means for controlling the processors of the computer system, such that the system, in assessing the target gene sequence and the reference sequences (41), aligns the target gene sequence and the reference sequences (41) associated with the selected sequence profile (42); and identifies the new informative sequence regions by identifying nucleotide codes corresponding at a same sequential position in at least a defined number of the target gene sequence and reference sequences (41).

24. The computer program product according to one of claims 19 to 23, comprising further computer program code means for controlling the processors of the computer system, such that the system determines the organism type-specific sequence profiles (42) stored in the database (4) by aligning organism type-specific gene sequences, by creating consensus sequences per organism type, by identifying the informative regions that enable differentiating the individual organism types, and by defining the organism type-specific sequence profiles (42) based on the informative regions.

25. The computer program product according to claim 24, comprising further computer program code means for controlling the processors of the computer system, such that the system includes in the organism type-specific sequence profiles (42), stored in the database (4), genus-specific or group-specific sequence profiles (42); and determines the genus-specific or group-specific sequence profiles (42) by aligning genus-specific or group-specific gene sequences, by creating consensus sequences per organism type, by identifying the informative regions that enable differentiating the individual organism types, and by defining the genus-specific or group-specific sequence profiles (42) based on the informative regions.

26. The computer program product according to one of claims 19 to 25, comprising further computer program code means for controlling the processors of the computer system, such that the system proofreads the target gene sequence based on the selected sequence profile (42) by comparing the target gene sequence to the reference sequences (41) associated with the selected sequence profile (42); assesses for differences of nucleotide codes, located in informative sequence regions, whether the differences indicate another organism type; and initiates adaptation of the selected sequence profile (42) for differences assessed to indicate another organism type.

27. The computer program product according to one of claims 19 to 26, comprising further computer program code means for controlling the processors of the computer system, such that the system receives the target gene sequence from a user via a telecommunications network (2); and transmits via the telecommunications network (2) to a user interface the organism type of the target gene sequence, defined by the organism type-specific reference sequence (41).

## Patentansprüche

1. Computerimplementiertes Verfahren zum Identifizieren von Organismustypen von einer Zielgensequenz, umfassend:
Empfangen der Zielgensequenz,
automatisch Auswählen aus einer Mehrzahl von Organismustyp-spezifischen Sequenzprofilen (42) in einer Datenbank (4), wobei jedes Sequenzprofil (42) informative Sequenzregionen zum Unterscheiden von einzelnen Organismustypen definiert und positionsspezifische Informationen umfasst, die mehr als einer von dazugehörigen Referenzsequenzen (41) eines spezifischen Organismustyps zugeordnet sind, eines ausgewählten Sequenzprofils (42), das die höchste Korrelation mit der Zielgensequenz aufweist, auf der Basis der positionsspezifischen Informationen des zugeordneten Organismustyp-spezifischen Profils,
automatisch Abfragen von Referenzsequenzen (41), die dem ausgewählten Sequenzprofil (42) zugehörig sind, von der Datenbank (4),
automatisch Vergleichen der Zielgensequenz mit den Referenzsequenzen (41) und automatisch Gewichten von Vergleichsergebnissen in Bezug auf die informativen Sequenzregionen unter Verwendung von Gewichtungsfaktoren, die den informativen Sequenzregionen zugehörig sind, die in dem ausgewählten Sequenzprofil definiert sind,
Bestimmen einer Organismustyp-spezifischen Referenzsequenz (41), welche die beste Übereinstimmung mit der Zielgensequenz aufweist, aus den Referenzsequenzen (41), wobei die beste Übereinstimmung auf der Basis der Vergleichsergebnisse bestimmt wird, die für die informativen Sequenzregionen gewichtet worden sind, und Zuordnen eines Organismustyps zu der Zielgensequenz auf der Basis der Organismustyp-spezifischen Referenzsequenz mit der besten Übereinstimmung.

2. Verfahren nach Anspruch 1, wobei die Vergleichsergebnisse eine Anzahl von Unterschieden der Nukleotidcodes von jeder der Referenzsequenzen (41) umfassen, wenn diese mit der Zielgensequenz verglichen werden, wobei das Gewichten der Vergleichsergebnisse das Bestimmen einer gewichteten Anzahl von Unterschieden durch Multiplizieren der Anzahl von Unterschieden, die den informativen Sequenzregionen zugeordnet sind, mit einem Gewichtungsfaktor für jede Referenzsequenz (41) umfasst, und wobei das Verfahren ferner das Speichern einer Liste der Referenzsequenzen (41) umfasst, wobei die Liste nach der gewichteten Anzahl von Unterschieden der jeweiligen Referenzsequenz (41) geordnet ist.

3. Verfahren nach Anspruch 1 oder 2, wobei die Vergleichsergebnisse eine Anzahl von Entsprechungen der Nukleotidcodes von jeder der Referenzsequenzen (41) umfassen, wenn diese mit der Zielgensequenz verglichen werden, wobei das Gewichten der Vergleichsergebnisse das Bestimmen einer gewichteten Anzahl von Entsprechungen durch Multiplizieren der Anzahl von Entsprechungen, die den informativen Sequenzregionen zugeordnet sind, mit einem Gewichtungsfaktor für jede Referenzsequenz (41) umfasst, und wobei das Verfahren ferner das Speichern einer Liste der Referenzsequenzen (41) umfasst, wobei die Liste nach der gewichteten Anzahl von Entsprechungen der jeweiligen Referenzsequenz (41) geordnet ist.

4. Verfahren nach einem der Ansprüche 1 bis 3, wobei die Zielgensequenz und die Referenzsequenzen (41), die dem ausgewählten Sequenzprofil (42) zugehörig sind, in Bezug auf neue informative Sequenzregionen für das ausgewählte Sequenzprofil (42) bewertet werden, und wobei das ausgewählte Sequenzprofil (42) durch Speichern einer neuen informativen Sequenzregion als Teil des ausgewählten Sequenzprofils (42) angepasst wird.

5. Verfahren nach Anspruch 4, wobei die Bewertung der Zielgensequenz und der Referenzsequenzen (41) das Ausrichten der Zielgensequenz und der Referenzsequenzen (41), die dem ausgewählten Sequenzprofil (42) zugehörig sind, und das Identifizieren der neuen informativen Sequenzregionen durch Identifizieren von Nukleotidcodes, die der gleichen Sequenzposition entsprechen, in mindestens einer definierten Anzahl der Zielgensequenz und der Referenzsequenzen (41) umfasst.

6. Verfahren nach einem der Ansprüche 1 bis 5, wobei die Organismustyp-spezifischen Sequenzprofile (42), die in der Datenbank (4) gespeichert sind, durch Ausrichten von Organismustyp-spezifischen Gensequenzen, durch Erzeugen von Konsensussequenzen pro Organismustyp, durch Identifizieren der informativen Regionen, die eine Unterscheidung der einzelnen Organismustypen ermöglichen, und durch Definieren der Organismustyp-spezifischen Sequenzprofile (42) auf der Basis der informativen Regionen bestimmt werden.

7. Verfahren nach Anspruch 6, wobei die Organismustyp-spezifischen Sequenzprofile (42), die in der Datenbank (4) gespeichert sind, genusspezifische oder gruppenspezifische Sequenzprofile (42) umfassen und wobei die genusspezifischen oder gruppenspezifischen Sequenzprofile (42) durch Ausrichten von genusspezifischen oder gruppenspezifischen Gensequenzen, durch Erzeugen von Konsensussequenzen pro Organismustyp, durch Identifizieren der informativen Regionen, die eine Unterscheidung der einzelnen Organismustypen ermöglichen, und durch Definieren der genusspezifischen oder gruppenspezifischen Sequenzprofile (42) auf der Basis der informativen Regionen bestimmt werden.

8. Verfahren nach einem der Ansprüche 1 bis 7, wobei die Zielgensequenz auf der Basis des ausgewählten Sequenzprofils (42) durch Vergleichen der Zielgensequenz mit den Referenzsequenzen (41), die dem ausgewählten Sequenzprofil (42) zugehörig sind, durch Bewerten in Bezug auf Unterschiede von Nukleotidcodes, die sich in informativen Sequenzregionen befinden, ob die Unterschiede einen weiteren Organismustyp anzeigen, und durch Initiieren einer Anpassung des ausgewählten Sequenzprofils (42) in Bezug auf Unterschiede, die so bewertet wurden, dass sie einen weiteren Organismustyp anzeigen, korrekturgelesen wird.

9. Verfahren nach einem der Ansprüche 1 bis 8, wobei die Zielgensequenz durch einen Server (3) von einem Benutzer mittels eines Telekommunikationsnetzwerks (2) empfangen wird und wobei der Organismustyp der Zielgensequenz, der durch die Organismustyp-spezifische Referenzsequenz (41) definiert ist, durch den Server (3) mittels des Telekommunikationsnetzwerks (2) zu einer Benutzerschnittstelle übertragen wird.

10. Computersystem zum Identifizieren von Organismustypen von einer Zielgensequenz, wobei das System umfasst:
eine Datenbank (4), die eine Mehrzahl von Organismustyp-spezifischen Sequenzprofilen (42) umfasst, wobei jedes Sequenzprofil (42) informative Sequenzregionen zum Unterscheiden von einzelnen Organismustypen umfasst und positionsspezifische Informationen umfasst, die mehr als einer von dazugehörigen Referenzsequenzen (41) eines spezifischen Organismustyps zugeordnet sind,
ein Profil (42)-Auswahlmodul (32), das zum automatischen Auswählen eines ausgewählten Sequenzprofils (42) aus den Sequenzprofilen (42), das die höchste Korrelation mit der Zielgensequenz aufweist, auf der Basis der positionsspezifischen Informationen des zugeordneten Organismustyp-spezifischen Profils ausgebildet ist,
ein Abfragemodul (33), das zum automatischen Abfragen von Referenzsequenzen (41), die zu dem ausgewählten Sequenzprofil (42) gehören, von der Datenbank (4) ausgebildet ist,
ein Vergleichsmodul (34), das zum automatischen Vergleichen der Zielgensequenz mit den Referenzsequenzen (41) und zum automatischen Gewichten von Vergleichsergebnissen, die den informativen Sequenzregionen zugeordnet sind, unter Verwendung von Gewichtungsfaktoren ausgebildet ist, die zu den informativen Sequenzregionen gehören, die in dem ausgewählten Sequenzprofil definiert sind,
ein Typbestimmungsmodul (35), das zum Bestimmen einer Organismustyp-spezifischen Referenzsequenz (41) von den Referenzsequenzen (41), welche die beste Übereinstimmung mit der Zielgensequenz aufweist, ausgebildet ist, wobei die beste Übereinstimmung auf der Basis der Vergleichsergebnisse bestimmt wird, die für die informativen Sequenzregionen gewichtet worden sind, und
Zuordnen eines Organismustyps zu der Zielgensequenz auf der Basis der Organismustyp-spezifischen Referenzsequenz mit der besten Übereinstimmung.

11. System nach Anspruch 10, wobei die Vergleichsergebnisse eine Anzahl von Unterschieden der Nukleotidcodes von jeder der Referenzsequenzen (41) umfassen, wenn diese mit der Zielgensequenz verglichen werden, wobei das Vergleichsmodul (34) zum Bestimmen einer gewichteten Anzahl von Unterschieden durch Multiplizieren der Anzahl von Unterschieden, die den informativen Sequenzregionen zugeordnet sind, mit einem Gewichtungsfaktor für jede Referenzsequenz (41) ausgebildet ist, und wobei das Typbestimmungsmodul (35) zum Speichern einer Liste der Referenzsequenzen (41) ausgebildet ist, wobei die Liste nach der gewichteten Anzahl von Unterschieden der jeweiligen Referenzsequenz (41) geordnet ist.

12. System nach Anspruch 10 oder 11, wobei die Vergleichsergebnisse eine Anzahl von Entsprechungen der Nukleotidcodes von jeder der Referenzsequenzen (41) umfassen, wenn diese mit der Zielgensequenz verglichen werden, wobei das Vergleichsmodul (34) zum Bestimmen einer gewichteten Anzahl von Entsprechungen durch Multiplizieren der Anzahl von Entsprechungen, die den informativen Sequenzregionen zugeordnet sind, mit einem Gewichtungsfaktor für jede Referenzsequenz (41) ausgebildet ist, und wobei das Typbestimmungsmodul (35) zum Speichern einer Liste der Referenzsequenzen (41) ausgebildet ist, wobei die Liste nach der gewichteten Anzahl von Entsprechungen der jeweiligen Referenzsequenz (41) geordnet ist.

13. System nach einem der Ansprüche 10 bis 12, das ferner ein Profil (42)-Anpassungsmodul (36) umfasst, das zum Bewerten der Zielgensequenz und der Referenzsequenzen (41), die dem ausgewählten Sequenzprofil (42) zugehörig sind, in Bezug auf neue informative Sequenzregionen für das ausgewählte Sequenzprofil (42) und zum Anpassen des ausgewählten Sequenzprofils (42) durch Speichern einer neuen informativen Sequenzregion als Teil des ausgewählten Sequenzprofils (42) ausgebildet ist.

14. System nach Anspruch 13, wobei das Profil (42)-Anpassungsmodul (36) zum Ausrichten der Zielgensequenz und der Referenzsequenzen (41), die dem ausgewählten Sequenzprofil (42) zugehörig sind, und zum Identifizieren der neuen informativen Sequenzregionen durch Identifizieren von Nukleotidcodes, die der gleichen Sequenzposition entsprechen, in mindestens einer definierten Anzahl der Zielgensequenz und der Referenzsequenzen (41) ausgebildet ist.

15. System nach einem der Ansprüche 10 bis 14, das ferner ein Profilierungsmodul (37) umfasst, das zum Bestimmen der Organismustyp-spezifischen Sequenzprofile (42), die in der Datenbank (4) gespeichert sind, durch Ausrichten von Organismustyp-spezifischen Gensequenzen, durch Erzeugen von Konsensussequenzen pro Organismustyp, durch Identifizieren der informativen Regionen, die eine Unterscheidung der einzelnen Organismustypen ermöglichen und durch Definieren der Organismustyp-spezifischen Sequenzprofile (42) auf der Basis der informativen Regionen ausgebildet ist.

16. System nach Anspruch 15, wobei die Organismustyp-spezifischen Sequenzprofile (42), die in der Datenbank (4) gespeichert sind, genusspezifische oder gruppenspezifische Sequenzprofile (42) umfassen und wobei das Profilierungsmodul (37) zum Bestimmen der genusspezifischen oder gruppenspezifischen Sequenzprofile (42) durch Ausrichten von genusspezifischen oder gruppenspezifischen Gensequenzen, durch Erzeugen von Konsensussequenzen pro Organismustyp, durch Identifizieren der informativen Regionen, die eine Unterscheidung der einzelnen Organismustypen ermöglichen und durch Definieren der genusspezifischen oder gruppenspezifischen Sequenzprofile (42) auf der Basis der informativen Regionen ausgebildet ist.

17. System nach einem der Ansprüche 10 bis 16, das ferner ein Korrekturlesemodul (38) umfasst, das zum Korrekturlesen der Zielgensequenz auf der Basis des ausgewählten Sequenzprofils (42) durch Vergleichen der Zielgensequenz mit den Referenzsequenzen (41), die dem ausgewählten Sequenzprofil (42) zugeordnet sind, zum Bewerten in Bezug auf Unterschiede von Nukleotidcodes, die sich in informativen Sequenzregionen befinden, ob die Unterschiede einen weiteren Organismustyp anzeigen, und zum Initiieren einer Anpassung des ausgewählten Sequenzprofils (42) in Bezug auf Unterschiede, die so bewertet wurden, dass sie einen weiteren Organismustyp anzeigen, ausgebildet ist.

18. System nach einem der Ansprüche 10 bis 17, das ferner ein Kommunikationsmodul (30) umfasst, das zum Empfangen der Zielgensequenz von einem Benutzer mittels eines Telekommunikationsnetzwerks (2) und zum Übertragen des Organismustyps der Zielgensequenz, der durch die Organismustyp-spezifische Referenzsequenz (41) definiert ist, mittels des Telekommunikationsnetzwerks (2) zu einer Benutzerschnittstelle ausgebildet ist.

19. Computerprogrammprodukt, das zur Durchführung des Verfahrens nach Anspruch 1 angepasst ist.

20. Computerprogrammprodukt nach Anspruch 19, das ferner Computerprogrammcodemittel zum Steuern der Prozessoren des Computersystems umfasst, so dass das System in die Vergleichsergebnissen bei einem Vergleich mit der Zielgensequenz eine Anzahl von Unterschieden bei den Nukleotidcodes von jeder der Referenzsequenzen (41) einbezieht, für jede Referenzsequenz (41) eine gewichtete Anzahl von Unterschieden durch Multiplizieren der Anzahl von Unterschieden, die den informativen Sequenzregionen zugeordnet sind, mit einem Gewichtungsfaktor bestimmt, und eine Liste der Referenzsequenzen (41) speichert, wobei die Liste nach der gewichteten Anzahl von Unterschieden der jeweiligen Referenzsequenz (41) geordnet ist.

21. Computerprogrammprodukt nach Anspruch 19 oder 20, das ferner Computerprogrammcodemittel zum Steuern der Prozessoren des Computersystems umfasst, so dass das System in die Vergleichsergebnisse eine Anzahl von Entsprechungen der Nukleotidcodes von jeder der Referenzsequenzen (41) einbezieht, wenn diese mit der Zielgensequenz verglichen werden, für jede Referenzsequenz (41) eine gewichtete Anzahl von Entsprechungen durch Multiplizieren der Anzahl von Entsprechungen, die den informativen Sequenzregionen zugeordnet sind, mit einem Gewichtungsfaktor bestimmt, und eine Liste der Referenzsequenzen (41) speichert, wobei die Liste nach der gewichteten Anzahl von Entsprechungen der jeweiligen Referenzsequenz (41) geordnet ist.

22. Computerprogrammprodukt nach einem der Ansprüche 19 bis 21, das ferner Computerprogrammcodemittel zum Steuern der Prozessoren des Computersystems umfasst, so dass das System die Zielgensequenz und die Referenzsequenzen (41), die dem ausgewählten Sequenzprofil (42) zugehörig sind, in Bezug auf neue informative Sequenzregionen für das ausgewählte Sequenzprofil (42) bewertet, und das ausgewählte Sequenzprofil (42) durch Speichern einer neuen informativen Sequenzregion als Teil des ausgewählten Sequenzprofils (42) anpasst.

23. Computerprogrammprodukt nach Anspruch 22, das ferner Computerprogrammcodemittel zum Steuern der Prozessoren des Computersystems umfasst, so dass das System bei der Bewertung der Zielgensequenz und der Referenzsequenzen (41) die Zielgensequenz und die Referenzsequenzen (41), die dem ausgewählten Sequenzprofil (42) zugehörig sind, ausrichtet und die neuen informativen Sequenzregionen durch Identifizieren von Nukleotidcodes, die der gleichen Sequenzposition entsprechen, in mindestens einer definierten Anzahl der Zielgensequenz und der Referenzsequenzen (41) identifiziert.

24. Computerprogrammprodukt nach einem der Ansprüche 19 bis 23, das ferner Computerprogrammcodemittel zum Steuern der Prozessoren des Computersystems umfasst, so dass das System die Organismustyp-spezifischen Sequenzprofile (42), die in der Datenbank (4) gespeichert sind, durch Ausrichten von Organismustyp-spezifischen Gensequenzen, durch Erzeugen von Konsensussequenzen pro Organismustyp, durch Identifizieren der informativen Regionen, die eine Unterscheidung der einzelnen Organismustypen ermöglichen und durch Definieren der Organismustyp-spezifischen Sequenzprofile (42) auf der Basis der informativen Regionen bestimmt.

25. Computerprogrammprodukt nach Anspruch 24, das ferner Computerprogrammcodemittel zum Steuern der Prozessoren des Computersystems umfasst, so dass das System in die Organismustyp-spezifischen Sequenzprofile (42), die in der Datenbank (4) gespeichert sind, genusspezifische oder gruppenspezifische Sequenzprofile (42) einbezieht und die genusspezifischen oder gruppenspezifischen Sequenzprofile (42) durch Ausrichten von genusspezifischen oder gruppenspezifischen Gensequenzen, durch Erzeugen von Konsensussequenzen pro Organismustyp, durch Identifizieren der informativen Regionen, die eine Unterscheidung der einzelnen Organismustypen ermöglichen und durch Definieren der genusspezifischen oder gruppenspezifischen Sequenzprofile (42) auf der Basis der informativen Regionen bestimmt.

26. Computerprogrammprodukt nach einem der Ansprüche 19 bis 25, das ferner Computerprogrammcodemittel zum Steuern der Prozessoren des Computersystems umfasst, so dass das System die Zielgensequenz auf der Basis des ausgewählten Sequenzprofils (42) durch Vergleichen der Zielgensequenz mit den Referenzsequenzen (41), die dem ausgewählten Sequenzprofil (42) zugehörig sind, korrekturliest, Unterschiede von Nukleotidcodes, die sich in informativen Sequenzregionen befinden, dahingehend bewertet, ob die Unterschiede einen weiteren Organismustyp anzeigen, und eine Anpassung des ausgewählten Sequenzprofils (42) in Bezug auf Unterschiede, die so bewertet wurden, dass sie einen weiteren Organismustyp anzeigen, initiiert.

27. Computerprogrammprodukt nach einem der Ansprüche 19 bis 26, das ferner Computerprogrammcodemittel zum Steuern der Prozessoren des Computersystems umfasst, so dass das System die Zielgensequenz von einem Benutzer mittels eines Telekommunikationsnetzwerks (2) empfängt und den Organismustyp der Zielgensequenz, der durch die Organismustyp-spezifische Referenzsequenz (41) definiert ist, mittels des Telekommunikationsnetzwerks (2) zu einer Benutzerschnittstelle überträgt.

## Revendications

1. Procédé mis en oeuvre sur ordinateur d'identification de types d'organisme depuis une séquence de gène cible, comprenant :
la réception d'une séquence de gène cible ;
la sélection automatique dans une base de données (4), parmi une série de profils de séquence spécifiques d'un type d'organisme (42), chaque profil de séquence (42) définissant des régions de séquence d'informations pour différencier les types individuels d'organisme et comprenant une information spécifique de position liée à plus d'une séquences de référence (41) associées d'un type spécifique d'organisme, le profil de séquence (42) sélectionné ayant la plus grande corrélation avec la séquence de gène cible sur base de ladite information spécifique de position du profil spécifique de type d'organisme apparenté ;
la récupération automatique dans la base de données (4), des séquences de référence (41) associées au profil de séquence (42) sélectionné ;
la comparaison automatique de la séquence de gène cible aux séquences de référence (41) et la pondération automatique des résultats de la comparaison liés aux régions de séquence d'informations en utilisant des facteurs de pondération associés aux régions de séquence d'informations définies dans le profil de séquence sélectionné ;
la détermination parmi les séquences de référence (41), d'une séquence de référence (41) spécifique d'un type d'organisme ayant la meilleure concordance avec la séquence de gène cible, la meilleure concordance étant déterminée sur base des résultats de la comparaison pondérés pour les régions de séquence d'informations, et
l'attribution à la séquence de gène cible, d'un type d'organisme sur base de la séquence de référence spécifique d'un type d'organisme ayant la meilleure concordance.

2. Procédé selon la revendication 1, où les résultats de la comparaison comprennent un certain nombre de différences dans les codes de nucléotides de chacune des séquences de référence (41) lors de la comparaison avec la séquence de gène cible ; où la pondération des résultats de la comparaison comprend la détermination pour chaque séquence de référence (41), d'un nombre pondéré de différences en multipliant par un facteur de pondération, le nombre de différences liées aux régions de séquence d'informations; et où le procédé comprend en outre, le stockage d'une liste de séquences de référence (41), la liste étant triée par le nombre pondéré de différences de la séquence de référence (41) respective.

3. Procédé selon la revendication 1 ou 2, où les résultats de la comparaison comprennent un nombre de correspondances dans les codes de nucléotides de chacune des séquences de référence (41) lors de la comparaison avec la séquence de gène cible ; où la pondération des résultats de la comparaison comprend la détermination pour chaque séquence de référence (41 ), d'un nombre pondéré de correspondances en multipliant par un facteur de pondération, le nombre de correspondances liées aux régions de séquence d'informations; et où le procédé comprend en outre, le stockage d'une liste de séquences de référence (41), la liste étant triée par le nombre pondéré de correspondances de la séquence de référence (41) respective.

4. Procédé selon l'une quelconque des revendications 1, à 3, où la séquence de gène cible et les séquences de référence (41) associées au profil de séquence (42) sélectionné sont évaluées pour de nouvelles régions de séquence d'informations pour le profil de séquence (42) sélectionné ; et où le profil de séquence (42) sélectionné est adapté en stockant une nouvelle région de séquence d'informations comme faisant partie du profil de séquence (42) sélectionné.

5. Procédé selon la revendication 4, où l'évaluation de la séquence de gène cible et des séquences de référence (41) comprend l'alignement de la séquence de gène cible et des séquences de référence (41) associées au profil de séquence (42) sélectionné, et l'identification des nouvelles régions de séquence d'informations par identification des codes de nucléotides correspondant à la même position séquentielle dans au moins un nombre défini de la séquence de gène cible et de séquences de référence (41).

6. Procédé selon l'une quelconque des revendications 1 à 5, où les profils de séquence (42) spécifiques d'un type d'organisme, stockés dans la base de données (4) sont déterminés par alignement de séquences de gène spécifiques d'un type d'organisme, en créant des séquences consensus par type d'organisme, en identifiant les régions d'informations qui permettent la différenciation des types d'organisme individuels, et en définissant les profils de séquence (42) spécifiques d'un type d'organisme sur base des régions d'informations.

7. Procédé selon la revendication 6, où les profils de séquence (42) spécifiques d'un type d'organisme stockés dans la base de données (4) comprennent des profils de séquence (42) spécifiques de genre ou spécifiques de groupe, et où les profils de séquence (42) spécifiques de genre ou spécifiques de groupe sont déterminés en alignant des séquences de gène spécifiques de genre ou spécifiques de groupe, en créant des séquences consensus par type d'organisme, en identifiant les régions d'informations qui permettent la différenciation des types d'organisme individuels, et en définissant profils de séquence (42) spécifiques de genre ou spécifiques de groupe sur base des régions d'informations.

8. Procédé selon l'une quelconque des revendications 1 à 7, où la séquence de gène cible est corrigée sur base du profil de séquence (42) sélectionné en comparant la séquence de gène cible aux séquences de référence (41) associées au profil de séquence (42) sélectionné, en évaluant les différences de codes de nucléotides, situées dans les régions de séquence d'informations, si les différences indiquent un autre type d'organisme et en initiant l'adaptation du profil de séquence (42) sélectionné concernant les différences évaluées pour indiquer un autre type d'organisme.

9. Procédé selon l'une quelconque des revendications 1 à 8, où la séquence de gène cible est réceptionnée par un serveur (3) depuis un utilisateur via un réseau de télécommunications (2) ; et où le type d'organisme de la séquence de gène cible défini par la séquence de référence (41) spécifique d'un organisme type, est transmis par le serveur (3) via le réseau de télécommunications (2) sur une interface de l'utilisateur.

10. Système d'ordinateur pour identifier des types d'organisme à partir d'une séquence de gène cible, le système comprenant :
une banque de données (4) comprenant une série de profils de séquence (42) spécifiques d'un type d'organisme, chaque profil de séquence (42) définissant des régions de séquence d'informations pour différencier des types d'organisme individuels et comprenant une information spécifique de position liée à plus d'une séquences de référence (41) associées d'un type d'organisme spécifique ;
un module de sélection (32) de profil (42) configuré pour sélectionner automatiquement parmi lesdits profils de séquence (42), un profil de séquence (42) sélectionné ayant la plus grande corrélation avec la séquence de gène cible sur base de la dite information spécifique de position du profil spécifique d'un type d'organisme apparenté ;
un module de récupération (33) configuré pour récupérer automatiquement dans la base de données (4), les séquences de référence (41) associées au profil de séquence (42) sélectionné ;
un module de comparaison (34) configuré pour comparer automatiquement la séquence de gène cible aux séquences de référence (41) et pondérer automatiquement les résultats de la comparaison liés aux régions de séquence d'informations en utilisant des facteurs de pondération associés aux régions de séquence d'informations définies dans le profil de séquence sélectionné ;
un module de détermination de type (35) configuré pour déterminé à partir des séquences de référence (41), une séquence de référence (41) spécifique d'un type d'organisme ayant la meilleure concordance avec la séquence de gène cible, la meilleure concordance étant déterminée sur base des résultats de la comparaison pondérés pour les régions de séquence d'informations, et
l'attribution à la séquence de gène cible, d'un type d'organisme sur base de la séquence de référence spécifique d'un type d'organisme ayant la meilleure concordance.

11. Système selon la revendication 10, où les résultats de la comparaison comprennent un nombre de différences dans les codes de nucléotides de chacune des séquences de référence (41) lors de la comparaison avec la séquence de gène cible ; où le module de comparaison (34) est configuré pour déterminer pour chaque séquence de référence (41), un nombre pondéré de différences en multipliant par un facteur de pondération, le nombre de différences liées aux régions de séquence d'informations ; et où le module de détermination de type (35) est configuré pour stocker une liste de séquences de référence (41), la liste étant triée par le nombre pondéré de différences de la séquence de référence (41) respective.

12. Système selon la revendication 10 ou 11, où les résultats de la comparaison comprennent un certain nombre de correspondances dans les codes de nucléotides de chacune des séquences de référence (41) lors de la comparaison avec la séquence de gène cible ; où le module de comparaison (34) est configuré pour déterminer pour chaque séquence de référence (41), un nombre pondéré de correspondances en multipliant par un facteur de pondération, le nombre de correspondances liées aux régions de séquence d'informations ; et où le module de détermination de type (35) est configurer pour stocker une liste de séquences de référence (41), la liste étant triée par le nombre pondéré de correspondances de la séquence de référence (41) respective.

13. Système selon l'une quelconque des revendications 10 à 12, comprenant en outre, un module d'adaptation (36) de profil (42) configuré pour évaluer la séquence de gène cible et les séquences de référence (41) associées au profil de séquence (42) sélectionné pour de nouvelles régions de séquence d'informations pour le profil de séquence (42) sélectionné, et pour adapter le profil de séquence (42) sélectionné en stockant une nouvelle région de séquence d'informations faisant partie du profil de séquence (42) sélectionné.

14. Système selon la revendication 13, où le module d'adaptation (36) de profil (42) est configuré pour aligner la séquence de gène cible et les séquences de référence (41) associées au profil de séquence (42) sélectionné, et pour identifier de nouvelles régions de séquence d'informations par identification des codes de nucléotides correspondant à la même position séquentielle dans au moins un nombre défini de la séquence de gène cible et de séquences de référence (41).

15. Système selon l'une quelconque des revendications 10 à 14, comprenant en outre, un module de profilage (37) configuré pour déterminer les profils de séquence (42) spécifiques d'un type d'organisme, stockés dans la base de données (4) en alignant les séquences de gène spécifiques d'un type d'organisme, en créant des séquences consensus par type d'organisme, en identifiant les régions d'informations qui permettent la différenciation des types d'organisme individuels, et en définissant les profils de séquence (42) spécifiques d'un type d'organisme sur base des régions d'informations.

16. Système selon la revendication 15, où les profils de séquence (42) spécifiques d'un type d'organisme stockés dans la base de données (4) comprennent des profils de séquence (42) spécifiques de genre ou spécifiques de groupe, et où le module de profilage (37) est configuré pour déterminer les profils de séquence (42) spécifiques de genre ou spécifiques de groupe en alignant des séquences de gène spécifiques de genre ou spécifiques de groupe, en créant des séquences consensus par type d'organisme, en identifiant les régions d'informations qui permettent la différenciation des types d'organisme individuels, et en définissant profils de séquence (42) spécifiques de genre ou spécifiques de groupe sur base des régions d'informations.

17. Système selon l'une quelconque des revendications 10 à 16, comprenant en outre, un module de correction (38) configuré pour corriger la séquence de gène cible sur base du profil de séquence (42) sélectionné en comparant la séquence de gène cible aux séquences de référence (41) associées au profil de séquence (42) sélectionné, pour évaluer les différences de codes de nucléotides, situées dans les régions de séquence d'informations, et si les différences indiquent un autre type d'organisme, et pour initier l'adaptation du profil de séquence (42) sélectionné pour les différences évaluées pour indiquer un autre type d'organisme.

18. Système selon l'une quelconque des revendications 10 à 17, comprenant en outre, un module de communication (30) configuré pour réceptionner la séquence de gène cible depuis un utilisateur via un réseau de télécommunications (2), et pour transmettre le type d'organisme de la séquence de gène cible, défini par la séquence de référence (41) spécifique d'un organisme type, via le réseau de télécommunications (2) sur une interface de l'utilisateur.

19. Programme d'ordinateur adaptée à réaliser le procédé de la revendication 1.

20. Programme d'ordinateur selon la revendication 19, comprenant en outre, des moyens de codage du programme d'ordinateur pour contrôler les processeurs du système d'ordinateur, de sorte que le système comprend dans les résultats de la comparaison, un nombre de différences au niveau des codes de nucléotides de chacune des séquences de référence (41) lors de la comparaison avec la séquence de gène cible ; détermine pour chaque séquence de référence (41), un nombre pondéré de différences en multipliant par un facteur de pondération, le nombre de différences liées aux régions de séquence d'informations ; et stocke une liste de séquences de référence (41), la liste étant triée par le nombre pondéré de différences de la séquence de référence (41) respective.

21. Programme d'ordinateur selon l'une des revendications 19 et 20, comprenant en outre, des moyens de codage du programme d'ordinateur pour contrôler les processeurs du système d'ordinateur, de sorte que le système comprend dans les résultats de la comparaison, un nombre de correspondances au niveau des codes de nucléotides de chacune des séquences de référence (41) lors de la comparaison avec la séquence de gène cible; détermine pour chaque séquence de référence (41), un nombre pondéré de correspondances en multipliant par un facteur de pondération, le nombre de correspondances liées aux régions de séquence d'informations ; et stocke une liste de séquences de référence (41), la liste étant triée par le nombre pondéré de correspondances de la séquence de référence (41) respective.

22. Programme d'ordinateur selon l'une quelconque des revendications 19 à 21, comprenant en outre, des moyens de codage du programme d'ordinateur pour contrôler les processeurs du système d'ordinateur, de sorte que le système évalue la séquence de gène cible et les séquences de référence (41) associées au profil de séquence (42) sélectionné pour de nouvelles régions de séquence d'informations pour le profil de séquence (42) sélectionné ; et adapte le profil de séquence (42) sélectionné en stockant une nouvelle région de séquence d'informations comme faisant partie du profil de séquence (42) sélectionné.

23. Programme d'ordinateur selon la revendication 22, comprenant en outre, des moyens de codage du programme d'ordinateur pour contrôler les processeurs du système d'ordinateur, de sorte que le système, dans l'évaluation de la séquence de gène cible et des séquences de référence (41), aligne la séquence de gène cible et les séquences de référence (41) associées au profil de séquence (42) sélectionné ; et identifie les nouvelles régions de séquence d'informations en identifiant les codes de nucléotides correspondant à la même position séquentielle dans au moins un nombre défini de la séquence de gène cible et des séquences de référence (41).

24. Programme d'ordinateur selon l'une quelconque des revendications 19 à 23, comprenant en outre, des moyens de codage du programme d'ordinateur pour contrôler les processeurs du système d'ordinateur, de sorte que le système détermine les profils de séquence (42) spécifiques d'un type d'organisme stockés dans la base de données (4) en alignant les séquences de gène spécifiques d'un type d'organisme, en créant des séquences consensus par type d'organisme, en identifiant les régions d'informations qui permettent la différenciation des types d'organisme individuels, et en définissant les profils de séquence (42) spécifiques d'un type d'organisme sur base des régions d'informations.

25. Programme d'ordinateur selon la revendication 24, comprenant en outre, des moyens de codage du programme d'ordinateur pour contrôler les processeurs du système d'ordinateur, de sorte que le système comprend dans les profils de séquence (42) spécifiques d'un type d'organisme, stockés dans la base de données (4), des profils de séquence (42) spécifiques de genre ou spécifiques de groupe, et détermine les profils de séquence (42) spécifiques de genre ou spécifiques de groupe en alignant des séquences de gène spécifiques de genre ou spécifiques de groupe, en créant des séquences consensus par type d'organisme, en identifiant les régions d'informations qui permettent la différenciation des types d'organisme individuels, et en définissant profils de séquence (42) spécifiques de genre ou spécifiques de groupe sur base des régions d'informations.

26. Programme d'ordinateur selon l'une quelconque des revendications 19 à 25, comprenant en outre, des moyens de codage du programme d'ordinateur pour contrôler les processeurs du système d'ordinateur, de sorte que le système corrige la séquence de gène cible sur base du profil de séquence (42) sélectionné en comparant la séquence de gène cible aux séquences de référence (41) associées au profil de séquence (42) sélectionné, évalue les différences de codes de nucléotides, situées dans les régions de séquence d'informations, et si les différences indiquent un autre type d'organisme ; et initie l'adaptation du profil de séquence (42) sélectionné concernant les différences évaluées pour indiquer un autre type d'organisme.

27. Programme d'ordinateur selon l'une quelconque des revendications 19 à 26, comprenant en outre, des moyens de codage du programme d'ordinateur pour contrôler les processeurs du système d'ordinateur, de sorte que le système réceptionne la séquence de gène cible depuis un utilisateur via un réseau de télécommunications (2), et transmet le type d'organisme de la séquence de gène cible, défini par la séquence de référence (41) spécifique d'un organisme type, via le réseau de télécommunications (2) sur une interface de l'utilisateur.
